# EUROPEAN PATENT APPLICATION

(11) **EP 4 643 785 A1**
(43) Date of publication of application: **05.11.2025**
(21) Application number: 25172232.8
(22) Date of filing: 24.04.2025
(51) Int. Cl.: A61B 8/08, A61B 8/00

(54) **QUANTITATIVE ULTRASOUND MEDICAL IMAGING ENHANCED BY INTERVENING TISSUE DETERMINATION**

(30) Priority: 29.04.2024 US 202418648959
(71) Applicant: Siemens Medical Solutions USA, Inc., Malvern, PA 19355 (US)
(72) Inventor: GUENETTE, Gilles D., Fall City, WA, 98024 (US); LABYED, Yassin, Carlsbad, CA, 92009 (US)
(74) Representative: HKW Intellectual Property PartG mbB

(57) **Abstract**

In one approach, quantitative ultrasound imaging is altered to account for the effects of intervening tissue (210). The tissue layers between the transducer (320) and the region (250) are measured, and the measurement is input to a machine-learned model (355) to quantify (140) from signals for the region of interest (250) and the measurement. This may provide more accurate quantification. In another approach, the region of interest (250) (ROI) is automatically placed (120), such as through detection (110) of anatomy (e.g., liver capsule), identification of and/or guidance (100) to the field of view, and/or scoring of imaging of the anatomy. This ROI placement (120) may avoid variability in quantification.

## Description

### BACKGROUND

The present embodiments relate to ultrasound imaging of soft tissue. In ultrasound imaging of soft tissue, such as liver imaging, a characteristic of the soft tissue may be diagnostically useful. Such characteristics include the elasticity, shear transmission, and/or fat fraction. The quantitative evaluation of tissue properties is influenced by the type, thickness, and alignment of the intervening tissue layers. For example, attenuation in the intervening layers may cause miscalculation of backscatter in the soft tissue. For ultrasound derived fat fraction (UDFF), the values for higher fat content may deviate from those determined using magnetic resonance. At higher fat, the ultrasound derived fat fraction may plateau or not rise as fast as magnetic resonance derived fat fraction, resulting in less accurate quantification.

Guidelines recommend, in the case of elastography, positioning a line for measurement from the skin surface to the measurement region of interest at a right angle to the liver capsule and to position the region of interest a minimum of 1 cm beneath the capsule. There is workflow difficulty in measuring angles and distances from a region of interest, such as measuring a distance from the region of interest to a liver capsule in a fat fraction measurement. Manual measurement or visual approximation of distances and angles is often used, leading to undesired variability in quantification.

### SUMMARY

By way of introduction, the preferred embodiments described below include methods, instructions, and systems for ultrasound imaging with an ultrasound scanner. In one approach, quantitative imaging is altered to account for the effects of intervening tissue. The tissue layers between the transducer and the region are measured, and the measurement is input to a machine-learned model to quantify from signals for a region of interest and the measurement. This may provide more accurate quantification. In another approach, the region of interest (ROI) is automatically placed, such as through detection of anatomy (e.g., liver capsule), identification of and/or guidance to the field of view, and/or scoring of imaging of the anatomy. This ROI placement may avoid variability in quantification.

In a first aspect, a method is provided for ultrasound imaging with an ultrasound scanner. The ultrasound scanner measures tissue between a liver and a transducer of the ultrasound scanner and scans a region of interest in the liver. An ultrasound derived fat fraction (UDFF) of the liver is determined using a first machine-learned model configured to receive the measurement of the tissue and information from the scanning and output the UDFF. The UDFF is displayed.

In a second aspect, a system is provided for ultrasound medical imaging. A beamformer is configured to scan soft tissue in a patient with the transducer. The soft tissue is in a region of interest. The beamformer is configured to scan intervening tissue between the transducer and the soft tissue with the transducer. An image processor is configured to position the region of interest and configured, by a first machine-learned model, to quantify a first characteristic of the soft tissue from the scan of the soft tissue. The first characteristic is an ultrasound derived fat fraction, shear wave characteristic, and/or elastography characteristic. The first machine-learned model receives a second characteristic of the intervening tissue as input to output the first characteristic. A display is configured to display an ultrasound image showing the quantification of the first characteristic of the soft tissue.

In a third aspect, a method is provided for ultrasound quantification of soft tissue characteristic with an ultrasound scanner. An image processor detects anatomy for positioning a region of interest in soft tissue. A shear, elasticity, and/or fat fraction of the soft tissue in the region of interest is determined. The shear, elasticity, and/or fat fraction of the region tissue in the region of interest is displayed.

Further aspects are summarized below in the Illustrative Embodiments. The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figure 1 is a flow chart diagram of one embodiment of a method for ultrasound imaging with an ultrasound scanner with measurement of intervening tissue incorporated into quantification and consistent ROI placement;
Figure 2 illustrates an ultrasound image for fat fraction quantification; and
Figure 3 is a block diagram of one embodiment of a system for ultrasound medical quantification accounting for intervening tissue and/or consistent ROI placement.

### DETAILED DESCRIPTION OF THE DRAWINGS AND PRESENTLY PREFERRED EMBODIMENTS

In one approach, tissue layer detection and modeling enhance quantitative ultrasound imaging. Intervening tissue layers are detected. The thickness or other measure of the intervening layers are used as part of the model for estimating tissue properties (quantification). In UDFF, measurements of one or more of the tissue layers between the liver capsule and the transducer are integrated into the model for evaluating the tissue property (i.e., fat fraction). The model uses input of the measurements with the signals or other information from the ROI to quantify.

For incorporation of the measurement of the intervening layers into the model for quantification (e.g., UDFF model), the modeling accuracy is improved. For example, when the indicator is placed on the liver capsule, the thickness of the intervening tissue layers is determined. This thickness can be factored into the model for assessing tissue properties, like in the UDFF model, to account for losses and/or wave distortions.

In a further approach, the ROI is placed automatically. The field of view may be guided, to then place the ROI at the appropriate location in a desired field of view. To enhance the workflow and eliminate the need to measure distances from the region of interest (ROI) to the capsule or the angle to the entrance, a liver-capsule indicator (e.g., "T" or "+") is incorporated into the ROI placement. The liver capsule indicator is positioned manually or automatically on or along the liver capsule. Automatic placement, such as by anatomy detection, reduces variability. This liver capsule indicator is used with an integrated region of interest to define the region to be quantified. The region is scanned for quantification. The liver capsule indicator supports visual alignment and improves confidence in placement of the measurement ROI. The indicator simplifies the workflow and increases confidence in the measurement or quantification of the liver (e.g., UDFF). In addition, consistent placement of the measurement ROI eliminates the variability associated with ROI positioning.

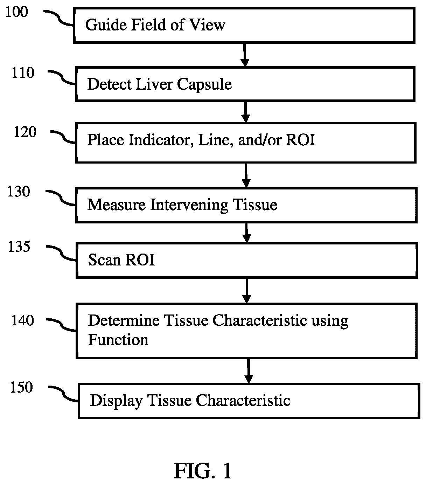

Figure 1 shows one embodiment of a method for ultrasound imaging with an ultrasound scanner. Ultrasound quantification, such as fat fraction or elasticity, is calculated. The calculation incorporates one or more measurements of the intervening layers of tissue into the calculation by artificial intelligence (AI) for a region of interest. The modeling of the quantification of a soft tissue characteristic is improved by considering the effects of the intervening tissue in the quantification through input to the AI. Alternatively, or additionally, the image processor consistently properly positions the ROI through field of view guidance and/or anatomy detection. Anatomy spaced from the ROI, such as liver capsule, may be detected. The user may be guided to properly position the field of view to then better position the ROI.

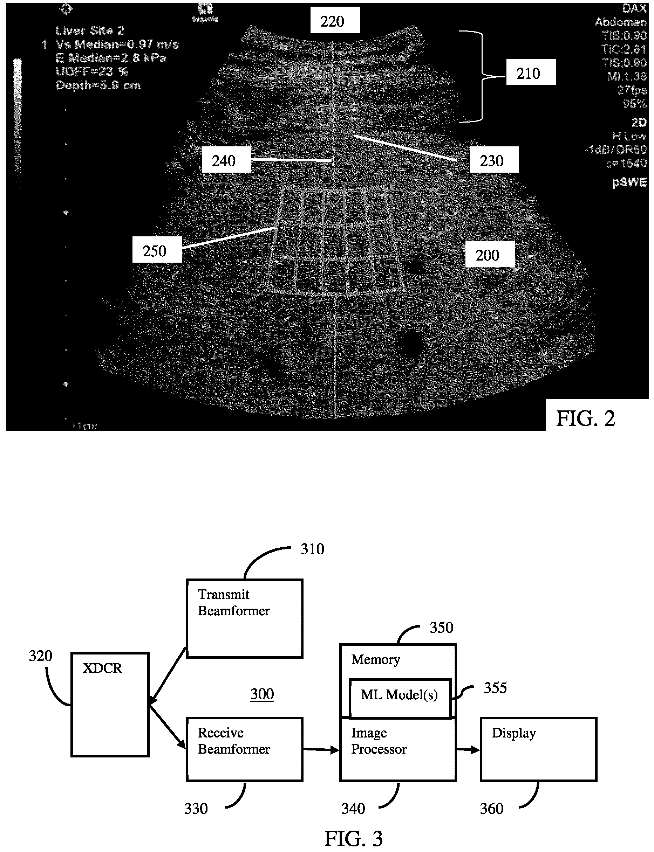

The method is implemented by the system of Figure 3 or a different system. A medical diagnostic ultrasound scanner performs measurements by acoustically generating the waves and measuring the responses with a beamformer. An image processor of the scanner, computer, server, or other device guides field of view placement, detects anatomy, places an indicator, line, or ROI, determines the soft tissue property or characteristic (e.g., fat fraction, elasticity, or shear wave speed), and generates an image of the quantification of the soft tissue characteristic. A display device, network, or memory is used to output the quantification image.

Additional, different, or fewer acts may be provided. For example, acts 100, 110, and/or 120 are not provided where AI-based quantification using information from the intervening tissue is used without field of view guidance and/or automated ROI placement. As another example, automated ROI placement is provided, such as using acts 100, 110, and/or 120 without using AI-based quantification in act 140 and/or using measurement of the intervening tissue in act 130. The acts are performed in the order described or shown (e.g., top to bottom or numerically), but may be performed in other orders.

In act 100, an image processor guides placement of the field of view of the ultrasound scanner and transducer. The transducer is positioned against the skin of the patient. The sonographer may then tilt, rotate, and/or translate the transducer to scan different parts of the patient. The image processor guides the sonographer to position the field of view at the desired soft tissue, such as the liver.

Various soft tissue may be of interest, such as the liver, kidneys, or another organ. The image processor may guide by detecting a current field of view and/or by scoring whether the current field of view is the desired field of view for a given diagnostic examination. For example, fat fraction is to be determined for the liver, so the field of view should include the liver imaged from a desired perspective. The orientation of the liver to the transducer may be important, such as placing the liver capsule perpendicular to a line from the center of the transducer. The image processor guides the sonographer to move the transducer to provide the desired field of view.

In one approach, the guidance is by feedback for the current field of view. The field of view is examined to determine a quality score or other indication of whether the field of view is proper. The user than alters the field of view by moving the transducer until the desired field of view is detected.

In another approach, the guidance is by feedback on how to adjust to have a better field of view. The image processor indicates how (e.g., rotation clockwise) and/or how much (e.g., 5 degrees) to move the transducer. For example, the user is guided to rock the transducer by X degrees in a given direction.

The image processor guides by examination of the current field of view. An ultrasound image or images, such as B-mode image, are input. Template matching, pattern matching, position sensing, detection, segmentation, and/or other image processing may be used to identify the anatomy represented in the image. This information may be used to score and/or to determine change to be made.

In one approach, a machine-learned model examines the field of view and outputs the feedback (e.g., quality/score for a current field of view and/or change to be made for a next field of view). The machine-learned model was trained with collected samples from sonographers seeking out and placing the field of view as desired for the diagnostic purpose (e.g., fat fraction determination). These samples include one or more images from the searching, the desired final image, and vectors indicating movement or change of the transducer to reach the desired position. Machine learning uses optimization to learn values for learnable parameters of the model, such as a neural network, to receive inputs (e.g., ultrasound images) and generate desired output (ground truth such as a score or other quality for the current field of view and/or a change to reach the desired field of view). For indicating how and how much to move the transducer, the machine-learned model may be a policy learned by reinforcement learning. The policy may be implemented by a neural network including a memory, such as long term short term memory. The inputs for training are a sequence, and the policy learns what action to take to progress to the desired field of view in the shortest time or fewest number of steps. For scoring, the machine-learned model may be a neural network, such as a convolutional neural network or a fully connected neural network for outputting a score based on ground truth scores curated by experts used in training.

Once trained, the machine-learned model outputs the quality (e.g., score) and/or change based on input of the current image or recent sequence of images. The current field of view is examined by the machine-learned model. The model itself outputs guidance or output from the model is used to generate an output guidance to position the transducer to image the soft tissue (e.g., liver) of interest.

The guidance may include reduction of the number or amount of shadowing and/or vessels in the field of view. For soft tissue quantification, shadowing and vessels near the region of interest are not desired. Shadowing caused by bones or other dense matter in the patient may be reduced by moving the transducer (i.e., by repositioning the field of view). The machine-learned model may specifically detect and/or score shadowing and/or the amount of fluid (e.g., number of vessels) and provide guidance to reduce them. Alternatively, the machine-learned model may score, where the score of quality is, in part, based on shadowing and/or vessels. For example, the samples may have ground truth fields of view (desired fields of view) where the transducer was moved to avoid fields of view with shadows and/or vessels. The machine-learned model is trained to score poorly and/or indicate change when the field of view is undesired, such as when shadows and/or vessels are in the field of view.

In some approaches, the field of view is identified by the machine-learned model, and guidance for placement of the transducer is provided to provide a quality image of the anatomy of interest (e.g., to limit shadows and/or vessels in the field of view). In other approaches for fat fraction, the machine-learned model identifies a liver capsule to guide placement of the transducer so the liver capsule is positioned as desired in the field of view (e.g., in the center 1/3 of the field of view with the border perpendicular to a line that is perpendicular to a center of the transducer).

Once the field of view is properly positioned, with or without guidance from image processor-based examination of the field of view, the ROI is positioned in act 120. The anatomy of interest, such as soft tissue (e.g., liver) is scanned for determining where to perform the quantification. The ROI is to be positioned in the field of view. The ultrasound medical scanner scans the tissue of the patient. The beamformer transmits acoustic beams and/or forms receive beams from acoustic echoes. An array of elements of the transducer transduces between acoustic and electrical energies. After B-mode detection, the signals from the acoustic echoes are scan converted for imaging. B-mode images are displayed for placement of the ROI for quantification.

The ROI may be placed manually by the sonographer. The ROI designates the region of the soft tissue of interest for which the soft tissue characteristic is to be determined.

In another approach, the ROI placement is assisted. For example, an image processor detects the location of anatomy of interest in act 110. This location may be highlighted to assist in ROI placement. For UDFF, the anatomy of interest is the liver capsule, which is to be positioned by movement of the transducer to be near a center laterally of the ultrasound images, such as within a center 1/3 of the field of view. The liver capsule is also to be substantially horizontal and perpendicular to a line perpendicular from the center of the transducer. Substantially accounts for +/- 10 degrees. The liver capsule is a border of the liver so is to be substantially perpendicular to a line from the transducer through the center of the liver capsule.

The liver capsule and/or other anatomy is detected by pattern or template matching, filtering and thresholding, region shrinking, skeletonization, and/or by AI. For example, a machine-learned model is trained with many samples from past patients curated by experts to detect the liver capsule in a B-mode image of the liver. The location of the liver capsule is automatically detected in the ultrasound image of the liver.

Figure 2 shows an example. An ultrasound image 200 of the liver is shown. The liver capsule is automatically detected. An indicator 230 is placed on the liver capsule as detected. The liver capsule is at a boundary between the liver and the transducer 220. One or more layers of intervening tissue 210 are between the liver capsule and the transducer 220. These layers of intervening tissue 210 may be identified and differentiated, allowing the system to better understand the acoustic properties and boundaries of each layer.

In act 120, the image processor automatically places a line 240 from the transducer 220 through the liver capsule, the indicator 230 on the liver capsule, and/or the ROI 250 in the liver along the line 240. The size of the ROI 250 and/or distance from the liver capsule may be set (e.g., 1 cm) by default or based on detection of other tissue. The line 240 is from a center of the transducer 220 through the liver capsule. The goal is to have the line be perpendicular to the liver capsule and the transducer 220. The ROI 250 is positioned within the liver, spaced from the liver capsule, such as by 1 cm or set other distance. The user may alter the position of the transducer 220, line 240, indicator 230, and/or ROI.

The automated detection of the liver capsule and placement of the indicator 230, line 240, and/or ROI 250 based on the location of anatomy spaced from the ROI provides a real-time visualization tool to help clinicians determine where to quantify. This real-time visualization tool also helps clinicians identify and assess intervening tissue layers 210 during ultrasound examinations, providing valuable context and improving the overall accuracy of the system.

Imaging occurs while placing the ROI, measuring intervening tissue, and/or quantification. For example, B-mode imaging is performed to place and maintain a field of view of the transducer and ROI at the desired tissue. Once the field of view and ROI are at the desired location within the patient, the scanning for estimating the tissue characteristic is performed. The imaging continues while tissue characteristic is quantified. Alternatively, the imaging ceases while the tissue characteristic is quantified.

In act 130, the ultrasound scanner measures tissue between the ROI (e.g., liver) and the transducer 220 of the ultrasound scanner. The intervening tissue may be between a boundary of the soft tissue being quantified and the transducer, such as the intervening tissue from the ROI 250 or liver capsule to the transducer 220. The intervening tissue being measured or accounted for may or may not include some actual intervening layers, such as the skin. The intervening tissue may be all or just parts of the soft tissue and/or fluid through which echoes from the ROI pass.

The tissue layer(s) indicated by the boundary marker (e.g., liver capsule for fat fraction) enable the identification and differentiation of various tissue layers during ultrasound examinations, allowing the system to better understand the acoustic properties and boundaries of each layer. The tissue from the organ of interest to the transducer is measured.

The measurement is of any one or more characteristics of the intervening tissue. To measure, one or more tissue layers or the intervening tissue as a whole is detected and/or segmented. Filtering and thresholding, pattern or template matching, skeletonization, and/or AI detects and/or segments. Image processing or machine-learning-based models automatically segment and/or differentiate tissue layers, reducing the need for manual intervention and streamlining the estimation process. Alternatively, the detection of the liver capsule or other anatomy relative to the transducer is used without further detection and/or segmentation of the intervening tissue.

The measurement may be a thickness. The thickness of each layer of tissue and/or the thickness of the total intervening tissue is measured. The placement of the ROI and/or detection of anatomy relative to the transducer indicates the thickness. The thickness of intervening tissue layers is incorporated into estimations of tissue characteristics, allowing for more accurate calculations of UDFF or another characteristic by accounting for the effect of layer thickness on acoustic properties.

In another approach, the measurement is a backscatter coefficient and/or attenuation coefficient for the intervening tissue as a whole, for different layers, and/or for different locations (e.g., pixels or voxels). The backscatter coefficient and/or attenuation coefficient may be used to determine the loss (e.g., by attenuation) and/or wave distortion (e.g., by backscatter level) in the intervening tissue so that the quantification in the ROI is more accurate. For example, the level of backscatter through the intervening tissue may affect the level of attenuation in the ROI. Rather than using localized attenuation and/or backscatter in the ROI, the global attenuation and/or backscatter from the transducer to the tissue in the ROI may be considered.

As yet another approach, the image processor identifies the types of tissue in the intervening tissue layers. Different types of tissue (e.g., skin, fat, muscle, other organs) have different acoustic effects (e.g., different attenuation and/or backscatter). The measurement is of the type of tissue. A machine-learned model, view in the image in combination with a knowledge base (e.g., template matching or look-up), and/or other detection or segmentation is used to distinguish different tissue layers and corresponding type of tissue of each of the layers. The type of tissue may be used to look-up acoustic properties of that type of tissue from a database. The acoustic properties may be used as the measurement. The measurement is of the types of tissue, number of layers, thickness of each layer, acoustic characteristics of each layer, and/or other information layer-by-layer.

Once the ROI is placed in act 120, with or without measurement of the intervening tissue in act 130, the ultrasound scanner scans the ROI, such as scanning the part of the liver, in act 135. The scanning is configured for determining the tissue characteristic. For example, multiple scans for determining UDFF are performed. Scans for backscatter calculation, attenuation calculation, elasticity, and/or shear wave speed are performed.

In act 140, the image processor determines (estimates) the tissue characteristic of the tissue in the ROI of the patient. For example, the UDFF of the liver of the patient is determined. The elasticity, shear wave transmission (e.g., speed), or other tissue characteristic may be determined. The quantification is more than mere B-mode (intensity of echo) or Doppler mode (e.g., velocity, power, or variance of tissue movement), but instead represents a characteristic of the tissue itself. The fat fraction, shear transmission, or elasticity is determined. Different tissues respond to shear waves (rather than the ultrasound wave) differently, so the shear wave speed indicates a characteristic of the tissue.

The fat fraction will be used as an example herein. The fat fraction is specific to the patient. One or more characteristics of the patient are used to obtain the fat fraction for that patient. Some patients may have the same or similar fat fraction, but different patients may have different fat fractions. In one embodiment, the tissue is the liver of the patient. The fat fraction for the liver of the patient is obtained. The liver example is used herein. In other embodiments, the tissue is the kidney, bladder, breast, heart, muscle, or another soft tissue of the patient.

The determination is by scanning the patient. The fat fraction may be obtained from a magnetic resonance scan of the patient, such as measured with a magnetic resonance proton density fat fraction (MR-PDFF) scan. Other modalities may be used to measure or estimate the fat fraction. In another embodiment, the fat fraction is obtained from an ultrasound scan. The ultrasound scanner estimating or measuring the fat fraction may be more cost and time effective and more convenient.

For ultrasound estimation of the fat fraction of the tissue (i.e., UDFF), the ultrasound medical scanner determines the scatter and attenuation from scanning the tissue. Other combinations of quantitative ultrasound parameters may be used. The complexity of human tissue may be measured using multiple quantitative ultrasound parameters for accurate characterization of that tissue. For example, liver fat fraction is estimated using a multi-parametric approach that combines quantitative parameters extracted from the received signals of different wave phenomena, such as scattering and attenuation of longitudinal waves, propagation and attenuation of shear waves, and/or propagation and attenuation of on-axis waves from acoustic radiation force impulse (ARFI) excitation. U.S. Published Patent Application No. 2018/0289323 discloses estimation of fat fraction using ultrasound.

In one embodiment, liver fat fraction is estimated by transmitting and receiving a sequence of pulses to estimate scattering parameters and by transmitting and receiving a sequence of pulses to obtain shear wave parameters. The estimation may include transmitting and receiving a sequence of pulses to estimate parameters from axial displacements caused by acoustic radiation force impulses (ARFI). The parameters are estimated and combined to estimate the fat fraction. Other information may be included in the estimation of the fat fraction, such as non-ultrasound data (e.g., blood biomarker). A look-up table based on empirical study may be used to relate values of the various parameters to values of fat fraction. Alternatively, a machine-learned model is trained using samples from various patients of scatter, attenuation, shear wave speed, and/or other measurements for the ROI with ground truth fat fraction from magnetic resonance and/or biopsy. The machine-learned model outputs the UDFF in response to input of the measurements from the ROI.

Other inputs may be used, such as input of measurements from the intervening tissue. The machine-learned model is trained using optimization from the training data of samples of inputs (ROI and intervening tissue measurements) and the ground truth output. The training configures (e.g., by determining values of learnable parameters) the machine-learned model to generate the output (e.g., UDFF) in response to the input (e.g., signals or measurements from the ROI and signals or measurements from the intervening tissue). Information from scanning the intervening tissue is input, such as inputting types of tissue, thickness, backscatter coefficient, and/or attenuation coefficient as a whole or layer-by-layer. Information from scanning the ROI is input, such as inputting return signals, attenuation, shear wave speed, and/or backscatter.

The information from the ROI is the information from the liver used to calculate UDFF or other quantification (e.g., elasticity). The information from the intervening tissue is information that accounts for losses and/or wave distortions caused by the tissue, which may influence the quantification in the ROI. The machine-learned model is adaptive to various tissue types. By including the information from the intervening tissue in the training, the machine-learned model learned to automatically adjust for different tissue types and their acoustic properties, increasing the accuracy of estimations of the quantification for the ROI even when dealing with diverse and complex tissue structures. This compensation mechanism corrects for the impact of intervening layers on ultrasound signals, allowing for more accurate estimations of tissue acoustic properties (e.g., backscatter coefficient and attenuation used for fat fraction estimation).

In one approach, the training samples are from a database of patients. Other information may be included. For example, a database of common tissue types and acoustic properties is used to create samples by simulation of ultrasound. Commonly encountered tissue types and their respective acoustic properties are used, allowing for more accurate estimations.

The quantification may be for fat fraction, elasticity, shear, or another tissue characteristic of soft tissue in the region of interest. The quantification may be made by processor application of a machine-learned model. In response to input, such as characteristics of intervening tissue and characteristics of the ROI, the machine-learned model generates the output quantification (e.g., UDFF value).

For UDFF, an ultrasound scanner generates a measure of scattering in tissue from a scan of a patient. To measure the scatter, the ultrasound scanner scans the tissue with ultrasound. A sequence of transmit and receive events is performed to acquire the signals to estimate the quantitative ultrasound scatter parameters. The measure of scatter measures a tissue response to a longitudinal wave transmitted from an ultrasound scanner. The scattering or echo of the longitudinal wave impinging on the tissue is measured.

The scatter is more than an intensity of reflection (i.e., more than B-mode data). Any measure of scatter may be used, such as a spectral slope of a logarithm of frequency-dependent backscatter coefficient. For example, the attenuation coefficient is measured. The reference-phantom method is used, but other measures of the attenuation coefficient may be used. Acoustic energy has an exponential decay as a function of depth. A measure of acoustic intensity as a function of depth before or without depth gain correction is performed. To remove system effects, the measurement is calibrated based on measures of acoustic intensity as a function of depth in a phantom. The measurement may be subject to less noise by averaging over a one, two, or three-dimensional region. The beamformed samples or acoustic intensity may be converted to the frequency domain, and the calculation performed in the frequency domain.

The attenuation is measured as a slope of intensity as a function of depth. Other measures of attenuation may be used, such as attenuation of a shear wave over distance or time. Tissue displacement as a function of depth from an ARFI induced longitudinal wave may be used to find attenuation of the tissue. The amount of maximum displacement, displacement as a function of depth, and/or displacement as a function of time is used to calculate the attenuation. Other propagation measures may be used instead of or as the attenuation. For example, a measure of shear wave propagation or a measure of on-axis displacement (e.g., ARFI measure) is used.

The fat fraction may be determined from the attenuation and scatter or from other combinations of quantitative ultrasound information. Values for the attenuation or propagation and backscatter measurements are input to a machine-learned classifier or look-up table, which outputs values of the fat fraction. The machine-trained classifier provides a nonlinear model. The look-up table may provide a linear model. A predetermined or programmed function relates the input values to the output values. The function and/or weights used in the function may be determined experimentally. For example, the weights are obtained by a least square minimization using magnetic resonance-proton density fat fraction (MR-PDFF) values or biopsy values of fat fraction. Other approaches to measuring fat fraction by the ultrasound medical scanner may be used.

The tissue characteristics (e.g., UDFF) is determined for the ROI. One value may be determined for the entire ROI. In other approaches, separate determination is made for separate sub-regions. A field of values (e.g., UDFF values) distributed in the ROI are determined. Figure 2 shows a 5 x 3 sub-division of the ROI. A value is determined for each sub-division in the ROI of the organ (e.g., liver). Greater resolution may be used.

In act 150, the ultrasound medical scanner or processor (e.g., image processor of the medical scanner) generates an image of the determined quantification. The generated image is transmitted to a display, memory, or computer network. For display, the generated image is displayed on the display device to provide diagnostically useful quantification characterizing the tissue in the ROI.

The image is a graph, alphanumeric text, and/or representation of tissue. In one approach, the quantification or tissue characterization value is displayed as an annotation or numeric value overlaid on a B-mode image. The overlay includes a graphic for the ROI. In other approaches, the ROI is coded (e.g., colored or highlighted) by the quantity or quantities (e.g., UDFF values). For example, each sub-region is color coded based on a scale. This color coding highlights each sub-region according to the value for that sub-region. In yet another approach, the user selects a location in the ROI. The determined quantity for that selected location is displayed as text or a marker along a scale. The image is a display of the determined shear, elasticity, and/or fat fraction for the ROI.

Figure 3 shows one embodiment of a system 300 for ultrasound medical imaging. The system 300 implements the method of Figure 1 or other methods. The system 300 uses (1) measures of intervening tissue as input to AI for quantifying in an ROI, (2) automated placement of ROI, (3) automated placement of the liver capsule indicator, and/or (4) field of view guidance (e.g., via image scoring). Using any of these, consistent and/or accurate quantification of soft tissue (e.g., UDFF or elasticity) is performed.

The system 300 includes the transmit beamformer 310, a transducer 320, the receive beamformer 330, an image processor 340, a display 360, and a memory 350. Additional, different, or fewer components may be provided. For example, a user input is provided for user interaction with the system.

The system 300 is a medical diagnostic ultrasound imaging system. In alternative embodiments, the system 300 may include a computer, workstation, picture archiving and communications system (PACS) station, or other arrangement at a same location or distributed over a network for real-time or post-acquisition imaging.

The transmit and receive beamformers 310, 330 form a beamformer for scanning (e.g., transmit and receive operations) using the transducer 320. Sequences of pulses are transmitted, and responses are received based on operation or configuration of the beamformer. The beamformer scans for determining the quantification and imaging tissue.

The transmit beamformer 310 is an ultrasound transmitter, memory, pulser, analog circuit, digital circuit, or combinations thereof. The transmit beamformer 310 is operable to generate waveforms for a plurality of channels with different or relative amplitudes, delays, and/or phasing. Upon transmission of acoustic waves from the transducer 320 in response to the generated electrical waveforms, one or more beams are formed.

The transducer 320 is a 1-, 1.25-, 1.5-, 1.75- or 2-dimensional array of piezoelectric or capacitive membrane elements. The transducer 320 includes a plurality of elements for transducing between acoustic and electrical energies. Receive signals are generated in response to ultrasound energy (echoes) impinging on the elements of the transducer 320. The elements connect with channels of the transmit and receive beamformers 310, 330. Alternatively, a single element with a mechanical focus is used.

The receive beamformer 330 includes a plurality of channels with amplifiers, delays, and/or phase rotators, and one or more summers. Each channel connects with one or more transducer elements. The receive beamformer 330 is configured by hardware, firmware, or software to apply relative delays, phases, and/or apodization to form one or more receive beams in response to each imaging transmission. The receive beamformer 330 outputs data representing spatial locations using the receive signals. Relative delays and/or phasing and summation of signals from different elements provide beamformation.

In coordination with the transmit beamformer 310, the receive beamformer 330 generates data representing the field of view. By scanning the field of view with ultrasound, data (e.g., beamformed samples) is generated. By repeating the scanning, ultrasound data representing the field of view at different times is acquired. The transmit beamformer 310 and receive beamformer 330 are configured to scan soft tissue in a patient with the transducer 320. The field of view for scanning the soft tissue is established by the position of the transducer 320 relative to the patient. The transducer 320 may be moved to change the field of view until the desired soft tissue is located. The ROI may then be positioned in the soft tissue for scanning the soft tissue. Different scanning, such as different transmit and receive sequences, may be used for the ROI than for other locations. Similarly, different scanning may be used for segmented or identified intervening tissue regions. The scan sequences to make the measurement are performed. The scan sequences to quantify in the ROI are performed. For other locations in the field of view, B-mode scanning is performed.

The image processor 340 may include a B-mode detector, Doppler detector, and/or pulsed wave Doppler detector for detecting and processing information for display from the beamformed ultrasound samples. The image processor 340 may include a processor, which may implement the detector or may be provided separately from the detector. The processor is a control processor, general processor, digital signal processor, tensor processor, graphics processing unit, application specific integrated circuit, field programmable gate array, network, server, group of processors, combinations thereof, or other now known or later developed device for quantifying tissue characteristics from ultrasound data (e.g., beamformed data or detected data). The image processor 340 is configured by hardware, firmware, and/or software to perform any combination of one or more of the acts shown in Figure 1.

In one approach, the image processor 340 is configured to guide a field of view of the transducer 320 to position the region of interest. The image processor 340 may be configured by a machine-learned model 355. The model 355 was trained to receive an image for a current field of view and score the quality of the image. The quality may be indicative of viewing the organ from the desired perspective, number or amount of shadowing, and/or number of vessels in the field of view. By communicating the quality to the user, the user may move the transducer to locate the field of view with sufficient or best quality. In another approach, the machine-learned model 355 was trained (is configured) to indicate a movement and/or magnitude of movement to reposition the field of view given input of a current field of view or sequence of views.

In another approach, the image processor 340 is configured to position the region of interest. Given an image for the current field of view, the image processor 340 is configured to position an ROI in the field of view. Segmentation or detection of anatomy (e.g., organ of interest) may be used. Alternatively, a machine-learned model 355 was trained (is configured) to place the ROI given an input image. For UDFF, the image processor 340 is configured to automatically detect the liver capsule. The soft tissue of interest is the liver. The ROI is to be positioned in the liver relative to the liver capsule. The liver capsule is detected. A liver capsule indicator and/or line through the liver capsule may be placed by the image processor based on the location of the detected liver capsule. The ROI is placed relative to the liver capsule, such as centered laterally on the line from the transducer to the liver capsule and a default distance from the liver capsule. The image processor 340 automatically places the ROI based on the detected liver capsule. The user may adjust the placement. In alternative approaches, the ROI and/or liver capsule indicator are placed manually by the user.

In yet another approach, the image processor 340 is configured to quantify a characteristic of the soft tissue from the scan of the soft tissue. The ROI is scanned. The scan data and/or measures derived from the scan data (e.g., backscatter coefficient, attenuation coefficient, and/or shear wave speed) are used to quantify. The quantified characteristic is the UDFF, shear wave characteristic, and/or elastography characteristic. In one implementation, the image processor 340 is configured by a machine-learned model 355 trained to output a value for the characteristic in response to input, such as the scan data and/or measures from the ROI.

In a further implementation, the image processor 340 is configured by the machine-learned model 355 to output the quantification in response to input of the information from the ROI and information from intervening tissue. The image processor 340 identifies the intervening tissue and receives measurements for the intervening tissue. The measurements characterize the intervening tissue, such as type of tissue, acoustic property of the tissue, layers of tissue, thickness of tissue, attenuation of tissue, and/or backscatter of tissue. Measurements for one or more of these characteristics are input to the machine-learned model 355. Through the previous training of the model 355, the model 355 was trained to account for loss and/or wave distortion of the intervening tissue. This accounting does not directly calculate the loss and/or wave distortion. In other implementations, the loss and/or wave distortion is calculated or estimated and the loss or wave distortion measures are input. By accounting for acoustic effects of the intervening tissue (e.g., between the transducer and the liver capsule or between the transducer and the ROI), a more accurate quantification may be provided.

The processor 340 is configured to generate one or more images. For example, a B-mode, contrast agent, M-mode, flow, color mode, and/or another type of image is generated. The quantification (e.g., UDFF) may be presented as an overlay in the image. The quantification modulates the color at locations in the ROI or is an annotation on the image. The quantification is included in the image or displayed sequentially or substantially simultaneously. For example, a tissue property estimate image is displayed at a same time as the other image. A value or values of the fat fraction or other quantification map to display information. Where the fat fraction or other quantification is measured at different locations, the values may be generated as a color overlay in the region of interest in B-mode images. Shear wave velocity and fat fraction or other quantification data may be combined as a single overlay on one B-mode image. Alternatively, the value of the fat fraction or quantification is displayed as text or a numerical value adjacent or overlaid on a B-mode or shear wave imaging image.

The image processor 340 may be configured to generate other displays. For example, a shear wave velocity image is displayed next to a graph, text, or graphical indicators of the fat fraction or another quantification. The quantification information is presented for one or more locations of the region of interest without being in a separate two or three-dimensional representation, such as where the user selects a location, and the ultrasound scanner then presents the fat fraction for that location.

The image processor 340 operates pursuant to instructions stored in the memory 350 or another memory. The memory 350 is a non-transitory computer readable storage media. The instructions for implementing the processes, methods and/or techniques discussed herein are provided on the computer-readable storage media or memories, such as a cache, buffer, RAM, removable media, hard drive, or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing, and the like.

In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

The display 360 is a device, such as a CRT, LCD, projector, plasma, or other display, for displaying one or two -dimensional images or three-dimensional representations. The two-dimensional images represent spatial distribution in an area. The three-dimensional representations are rendered from data representing spatial distribution in a volume. The display 360 is configured by the image processor 340 or other device by input of the data to be displayed as an image. The display 360 displays an image representing the tissue, such as the liver. The display 360 is configured to display the ultrasound image showing the quantification of the characteristic of the soft tissue in the ROI.

Various Illustrative Embodiments are summarized below. The combinations shown or other combinations may be provided. Aspects, functions, or approaches in one type (e.g., method or system) of illustrative embodiment may be used in another type (e.g., system or method). Any of the aspects, functions, or approaches may be used in a computer program product or instructions stored on a non-transitory computer readable storage medium.

Illustrative Embodiment 1: a method for ultrasound imaging with an ultrasound scanner, the method comprising: measuring, by the ultrasound scanner, tissue between a liver and a transducer of the ultrasound scanner; scanning, by the ultrasound scanner, a region of interest in the liver; determining an ultrasound derived fat fraction (UDFF) of the liver using a first machine-learned model configured to receive the measurement of the tissue and information from the scanning and output the UDFF; and displaying the ultrasound derived fat fraction.

Illustrative Embodiment 2: the method of Illustrative Embodiment 1 wherein measuring comprises measuring a thickness as the measurement.

Illustrative Embodiment 3: the method of any of Illustrative Embodiments 1-2 wherein measuring comprises measuring a backscatter coefficient and/or attenuation of the tissue as the measurement, and wherein determining comprises determining where the information comprises the backscatter coefficient and/or attenuation of the liver.

Illustrative Embodiment 4: the method of any of Illustrative Embodiments 1-3 wherein measuring comprises measuring the tissue between a liver capsule of the liver and the transducer, wherein the liver capsule is indicated by an indicator on a display.

Illustrative Embodiment 5: the method of any of Illustrative Embodiments 1-4 wherein measuring comprises measuring an acoustic property based on a type of the tissue as the measurement.

Illustrative Embodiment 6: the method of any of Illustrative Embodiments 1-5 wherein measuring comprises identifying different tissue layers of the tissue, and wherein the measurement is derived from the different tissue layers.

Illustrative Embodiment 7: the method of Illustrative Embodiment 6 wherein a characteristic of each of the different tissue layers is input to the first machine-learned model as the measurement.

Illustrative Embodiment 8: the method of any of Illustrative Embodiments 1-7 wherein the machine-learned model is configured by training to account for losses and/or wave distortions caused by the tissue.

Illustrative Embodiment 9: the method of any of Illustrative Embodiments 1-8 further comprising automatically detecting a location of a liver capsule in an ultrasound image of the liver and automatically placing a line from the transducer through the liver capsule, an indicator on the liver capsule, and the region of interest in the liver along the line, and wherein determining the UDFF comprise determining the UDFF in the region of interest.

Illustrative Embodiment 10: the method of any of Illustrative Embodiments 1-9 further comprising examining a field of view by a second machine-learned model and outputting guidance to position the transducer to image the liver based on output of the second machine-learned model.

Illustrative Embodiment 11: the method of Illustrative Embodiment 10 wherein examining comprises examining for shadows and/or vessels, wherein the guidance reduces the shadows and/or vessels in the field of view.

Illustrative Embodiment 12: the method of Illustrative Embodiment 10 wherein examining comprises scoring the field of view for automated placement of the region of interest.

Illustrative Embodiment 13: the method of any of Illustrative Embodiments 1-12 wherein determining comprises determining the UDFF as a field of UDFF values distributed in a region of interest in the liver, and wherein displaying comprises displaying an ultrasound image with the region of interest coded by the UDFF values.

Illustrative Embodiment 14: a system for ultrasound medical imaging, the system comprising: a transducer; a beamformer configured to scan soft tissue in a patient with the transducer, the soft tissue being in a region of interest, and to scan intervening tissue between the transducer and the soft tissue with the transducer; an image processor configured to position the region of interest and configured, by a first machine-learned model, to quantify a first characteristic of the soft tissue from the scan of the soft tissue, the first characteristic comprising an ultrasound derived fat fraction, shear wave characteristic, and/or elastography characteristic, the first machine-learned model receiving a second characteristic of the intervening tissue as input to output the first characteristic; and a display configured to display an ultrasound image showing the quantification of the first characteristic of the soft tissue.

Illustrative Embodiment 15: the system of Illustrative Embodiment 14 wherein the first machine-learned model is configured, by training, to account for loss and/or wave distortion of the intervening tissue.

Illustrative Embodiment 16: the system of any of Illustrative Embodiments 14-15 wherein the image processor is configured by a second machine-learned model to guide a field of view of the transducer to position the region of interest.

Illustrative Embodiment 17: the system of any of Illustrative Embodiments 14-16 wherein the soft tissue comprises a liver, wherein the intervening tissue comprises tissue between a liver capsule and the transducer, and wherein the image processor is configured to automatically detect the liver capsule and automatically place the region of interest based on the detected liver capsule.

Illustrative Embodiment 18: a method for ultrasound quantification of soft tissue characteristic with an ultrasound scanner, the method comprising: positioning, by an image processor detection of anatomy, a region of interest in soft tissue; quantifying a shear, elasticity, and/or fat fraction of the soft tissue in the region of interest; and displaying the shear, elasticity, and/or fat fraction of the region tissue in the region of interest.

Illustrative Embodiment 19: the method of Illustrative Embodiment 18 wherein positioning comprises identifying a field of view by a first machine-learned model and guiding placement of a transducer to limit shadows and/or vessels in the field of view.

Illustrative Embodiment 20: the method of any of Illustrative Embodiments 18-19 wherein positioning comprises identifying a liver capsule by a first machine-learned model and guiding placement of a transducer so that the liver capsule is in a center third laterally of an ultrasound image of a liver and the liver capsule is substantially perpendicular to a line from a center of the transducer to the liver capsule.

Illustrative Embodiment 21: the method of any of Illustrative Embodiments 18-20 further comprising measuring a first characteristic of intervening tissue between a transducer of the ultrasound scanner and the region of interest, wherein quantifying comprises quantifying by a machine-learned model in response to input of the first characteristic.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A method for ultrasound imaging with an ultrasound scanner, the method comprising:
measuring (130), by the ultrasound scanner, tissue (210) between a liver and a transducer (320) of the ultrasound scanner;
scanning (135), by the ultrasound scanner, a region of interest (250) in the liver;
determining (140) an ultrasound derived fat fraction (UDFF) of the liver using a first machine-learned model (355) configured to receive the measurement of the tissue (210) and information from the scanning (135) and output the UDFF; and
displaying (150) the ultrasound derived fat fraction.

2. The method of claim 1 wherein measuring (130) comprises measuring (130) a thickness as the measurement.

3. The method of claim 1 or 2 wherein measuring (130) comprises measuring (130) a backscatter coefficient and/or attenuation of the tissue (210) as the measurement, and wherein determining (140) comprises determining (140) where the information comprises the backscatter coefficient and/or attenuation of the liver.

4. The method of one of claims 1 to 3 wherein measuring (130) comprises measuring (130) the tissue (210) between a liver capsule of the liver and the transducer (320), wherein the liver capsule is indicated by an indicator (230) on a display.

5. The method of one of claims 1 to 4 wherein measuring (130) comprises measuring (130) an acoustic property based on a type of the tissue (210) as the measurement.

6. The method of one of claims 1 to 5 wherein measuring (130) comprises identifying different tissue layers of the tissue (210), and wherein the measurement is derived from the different tissue layers.

7. The method of claim 6 wherein a characteristic of each of the different tissue layers is input to the first machine-learned model (355) as the measurement.

8. The method of one of claims 1 to 7 wherein the machine-learned model (355) is configured by training to account for losses and/or wave distortions caused by the tissue (210).

9. The method of one of claims 1 to 8 further comprising automatically detecting (110) a location of a liver capsule in an ultrasound image of the liver and automatically placing (120) a lines (240) from the transducer (320) through the liver capsule, an indicator (230) on the liver capsule, and the region of interest (250) in the liver along the lines (240), and wherein determining (140) the UDFF comprise determining (140) the UDFF in the region of interest (250).

10. The method of one of claims 1 to 9 further comprising examining a field of view by a second machine-learned model (355) and outputting (100) guidance to position the transducer (320) to image the liver based on output of the second machine-learned model (355).

11. The method of claim 10 wherein examining comprises examining for shadows and/or vessels, wherein the guidance reduces the shadows and/or vessels in the field of view.

12. The method of claim 10 wherein examining comprises scoring the field of view for automated placement of the region of interest (250).

13. The method of one of claims 1 to 12 wherein determining (140) comprises determining (140) the UDFF as a field of UDFF values distributed in a region of interest (250) in the liver, and wherein displaying (150) comprises displaying (150) an ultrasound image with the region of interest (250) coded by the UDFF values.

14. A system for ultrasound medical imaging, the system comprising:
a transducer (320);
a beamformer (310, 330) configured to scan soft tissue in a patient with the transducer (320), the soft tissue being in a region of interest (250), and to scan intervening tissue (210) between the transducer (320) and the soft tissue with the transducer (320);
an image processor (340) configured to position the region of interest (250) and configured, by a first machine-learned model (355), to quantify a first characteristic of the soft tissue from the scan of the soft tissue, the first characteristic comprising an ultrasound derived fat fraction, shear wave characteristic, and/or elastography characteristic, the first machine-learned model (355) receiving a second characteristic of the intervening tissue (210) as input to output the first characteristic; and
a display (360) configured to display an ultrasound image showing the quantification of the first characteristic of the soft tissue.

15. The system of claim 14 wherein the first machine-learned model (355) is configured, by training, to account for loss and/or wave distortion of the intervening tissue (210).

16. The system of claim 14 or 15 wherein the image processor (340) is configured by a second machine-learned model (355) to guide a field of view of the transducer (320) to position the region of interest (250).

17. The system of one of claims 14 to 16 wherein the soft tissue comprises a liver, wherein the intervening tissue (210) comprises tissue between a liver capsule and the transducer (320), and wherein the image processor (340) is configured to automatically detect the liver capsule and automatically place the region of interest (250) based on the detected liver capsule.

18. A method for ultrasound quantification of soft tissue characteristic with an ultrasound scanner, the method comprising:
positioning (120), by an image processor (340) detection of anatomy, a region of interest (250) in soft tissue;
quantifying (140) a shear, elasticity, and/or fat fraction of the soft tissue in the region of interest (250); and
displaying (150) the shear, elasticity, and/or fat fraction of the region tissue in the region of interest (250).

19. The method of claim 18 wherein positioning (120) comprises identifying a field of view by a first machine-learned model (355) and guiding (100) placement of a transducer (320) to limit shadows and/or vessels in the field of view.

20. The method of claim 18 wherein positioning (120) comprises identifying a liver capsule by a first machine-learned model (355) and guiding (100) placement of a transducer (320) so that the liver capsule is in a center third laterally of an ultrasound image of a liver and the liver capsule is substantially perpendicular to a lines (240) from a center of the transducer (320) to the liver capsule.

21. The method of one of claims 18 to 20 further comprising measuring (130) a first characteristic of intervening tissue (210) between a transducer (320) of the ultrasound scanner and the region of interest (250), wherein quantifying comprises quantifying by a machine-learned model (355) in response to input of the first characteristic.
